# FASCICULE DE BREVET EUROPEEN

(11) **EP 0 593 332 B1**
(45) Date de publication et mention de la délivrance du brevet: **14.01.1998**
(21) Numéro de dépôt: 93402424.1
(22) Date de dépôt: 04.10.1993
(51) Int. Cl.: C07F 5/02, C07D 257/04, C07D 403/10

(54) **Dérivés de l'acide benzéneborinique leur préparation et leur utilisation comme intermédiaires de synthèse**
Phenylborinsäure-Derivate, ihre Herstellung und ihre Verwendung als Zwischenprodukte bei der Synthese
Phenylborinic acid derivatives, their preparation and their use as intermediates in syntheses

(30) Priorité: 12.10.1992 FR 9212166
(43) Date de publication de la demande: 20.04.1994
(73) Titulaire: SYNTHELABO, 92350 Le Plessis Robinson (FR)
(72) Inventeur: Chekroun, Isaac, F-93800 Epinay (FR); Ruiz-Montes, José, F-78200 Mantes La Jolie (FR); Bedoya Zurita, Manuel, E-28020 Madrid (ES); Rossey, Guy, F-78960 Voisins le Bretonneux (FR)
(74) Mandataire: Thouret-Lemaitre, Elisabeth

(56) Documents cités:
- EP-A- 0 470 795
- EP-A- 0 504 888
- FR-A- 1 303 881
- US-A- 5 130 439
- US-A- 5 149 699

## Description

La présente invention concerne des dérivés de l'acide benzèneborinique répondant à la formule (1) 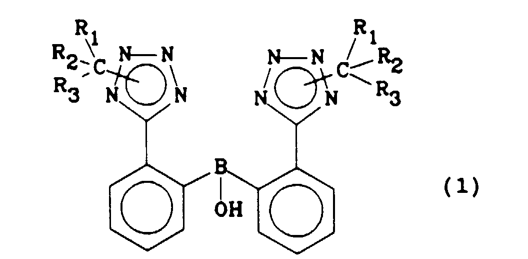 dans laquelle R₁, R₂ et R₃ représentent chacun indépendamment l'un de l'autre, soit un groupe (C₁-C₂)alkyle, soit un groupe aryle, le groupe -CR₁R₂R₃ étant en position 1 ou 2 du cycle tétrazole,
leur préparation et leur utilisation dans des couplages aryle-aryle, aryle-naphtyle ou aryle-hétérocycle en présence de catalyseurs au palladium.

Les composés préférés selon l'invention sont les composés répondant à la formule (1) dans laquelle le groupe -CR₁R₂R₃ est le groupe 1,1-diméthyléthyle.

Les composés de l'invention peuvent être préparés, à partir du 2-(1,1-diméthyléthyl)-5-phényltétrazole de la manière suivante : on fait réagir le 2-(1,1-diméthyléthyl)-5-phényltétrazole avec un alkyllithium, tel le butyllithium, dans un solvant aprotique comme le tétrahydrofurane, à une température comprise entre - 50 °C et + 66 °C. On obtient un organolithien que l'on fait réagir avec du trialkylborate dans un solvant tel que le tétrahydrofurane. On obtient un benzèneborinate d'alkyle que l'on soumet à une réaction d'hydrolyse.

Dans une variante du procédé, on prépare un organomagnésien à partir d'un dérivé halogéné du 2-(1,1-diméthyléthyl)-5-phényltétrazole puis on procède comme décrit ci-dessus.

Les composés de départ sont disponibles dans le commerce ou décrits dans la littérature ou peuvent être préparés selon des méthodes qui y sont décrites ou qui sont connues de l'homme du métier.

Ainsi, le 2-(1,1-diméthyléthyl)-5-phényltétrazole est préparé selon la méthode décrite pour un dérivé analogue par J. W. TILLEY et coll. *J. Med. Chem. 1991*, **34**, 1125-1126.

Les dérivés de l'acide benzèneborinique ainsi obtenus, sont des solides stables que l'on peut coupler avec des halogénures aromatiques ou hétérocycliques comportant de nombreux substituants tels que par exemple, les groupes carboxamide, carbonyle, carboxyle, cyano, alcoxyle ou nitro. Ces couplages peuvent être réalisés en milieu hydroorganique.

Le groupement 1,1-diméthyléthyle est un groupement protecteur particulièrement stable dans différentes conditions réactionnelles rencontrées en chimie organique. Son utilisation comme groupement protecteur de la fonction tétrazolyle, autorise l'emploi des composés de l'invention et des produits en résultant, dans différentes réactions de transformation chimique.

Les exemples qui suivent illustrent en détail la préparation des composés selon l'invention.
Les microanalyses et les spectres IR et RMN confirment la structure des composés obtenus.

### Exemple

### acide bis[2-[2-(1,1-diméthyléthyl)-2H-tétrazol-5-yl] phényl]borinique

### Voie organolithienne

Dans un ballon tricol de 1 litre, sous azote, on introduit 50 g (0,247 mole) de 2-(1,1-diméthyléthyl)-5-phényl-2*H*-tétrazole et 200 ml de tétrahydrofurane anhydre. On laisse refroidir la solution et on ajoute goutte à goutte, 150 ml d'une solution de n-butyllithium dans l'hexane. On agite le mélange pendant 30 minutes à la température ambiante puis pendant 30 minutes à 50 °C. On refroidit le milieu réactionnel à 0 °C et on ajoute une solution de 17 ml (0,148 mole) de triméthylborate dans 100 ml de tétrahydrofurane anhydre en maintenant la température à 0 °C. On laisse le mélange une nuit, sous agitation, à la température ambiante puis on le verse sur 250 ml d'une solution d'acide chlorhydrique à 10 %. On extrait le mélange par 500 ml d'acétate d'éthyle, on recueille la phase organique, on la lave par 100 ml d'eau et on la sèche sur du sulfate de magnésium. Après filtration et évaporation des solvants, on cristallise le produit dans l'éther isopropylique.
On obtient 24,3 g de produit.
Point de fusion = 157-159 °C Rendement = 45 %

### Voie organomagnésienne

Dans un ballon tricol de 100 ml muni d'un réfrigérant, sous azote, on introduit 0,37 g (15 mmoles) de magnésium en tournures dans 5 ml de tétrahydrofurane anhydre. On ajoute ensuite goutte à goutte, en maintenant un léger reflux, 3,28 g (10 mmoles) de 2-(1,1-diméthyléthyl)-5-(2-iodophényl)-2*H*-tétrazole dans 20 ml de tétrahydrofurane anhydre. On agite la solution pendant 30 minutes à 50 °C puis on laisse revenir à la température ambiante. On ajoute alors goute à goutte, à cette solution, 2,3 ml (20 mmoles) de triméthylborate dans 15 ml de tétrahydrofurane anhydre. On laisse le mélange une nuit, sous agitation, à la température ambiante puis on le verse sur 50 ml d'une solution d'acide chlorhydrique à 10 %. On extrait le mélange par 100 ml d'acétate d'éthyle, on recueille la phase organique, on la lave par 30 ml d'eau et on la sèche sur du sulfate de magnésium. Après filtration et évaporation des solvants, on purifie le résidu obtenu, par chromatographie sur colonne de gel de silice.
On obtient 1,4 g de produit.
Point de fusion = 157-159 °C Rendement = 65 %

Les composés selon l'invention peuvent être utilisés pour la synthèse de composés répondant à la formule (I) 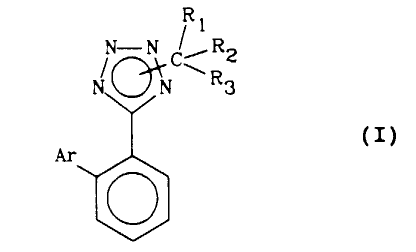 dans laquelle Ar représente soit un groupe aryle éventuellement substitué, soit un groupe naphtyle éventuellement substitué, soit un hétérocycle choisi parmi les groupes pyridinyle, pyrimidinyle, thiényle, imidazolyle, quinoléinyle et imidazopyridinyle éventuellement substitués et R₁, R₂ et R₃ sont tels que définis ci-dessus.
Des composés de formule (I) sont décrits dans les demandes de brevets européens nos 0500409 et 0540400 de la demanderesse.

La synthèse des composés de formule (I) à partir des composés de l'invention est réalisée selon la méthode décrite ci-après. On fait réagir un dérivé de l'acide benzèneborinique de formule générale (1) avec un dérivé de formule générale Ar-Z dans laquelle Z est un atome d'halogène et Ar est tel que défini ci-dessus, en présence d'une base telle que par exemple, le carbonate de sodium, le carbonate de potassium, le dihydrogènophosphate de potassium ou une amine tertiaire comme la triéthylamine et d'un catalyseur tel le palladiumtétrakis(triphénylphosphine), dans un solvant tel que par exemple, le toluène, le benzène, le xylène, le diméthylformamide ou un éther commme le 1,2-diméthoxyéthane.

Les exemples ci-dessous illustrent la synthèse des composés de formule générale (I) à partir des composés selon l'invention.

### A 6-butyl-2-(2-phényléthyl)-5-[[2'[2-(1,1-diméthyléthyl)-2H-tétrazol-5-yl][1,1'-biphényl]-4-yl]méthyl]-pyrimidin-4(3H)one

Dans un ballon bicol muni d'un réfrigérant, on introduit successivement 10,1 g (23,5 mmoles) d'acide bis[2-[2-(1,1-diméthyléthyl)-2*H*-tétrazol-5-yl]phényl] borinique, 20 g (47 mmoles) de 5-[(4-bromophényl)méthyl]-6-butyl-2-(2-phényléthyl)pyrimidin-4(3*H*)one, 1,63 g (1,41 mmoles) de palladium-tétrakis(triphénylphosphine), 47 ml d'une solution de carbonate de sodium 2 M et 200 ml de toluène. On porte ce mélange à la température de reflux pendant 16 heures. Après refroidissement et décantation, on recueille la phase aqueuse et on l'extrait avec 300 ml d'acétate d'éthyle. On rassemble les phases organiques et on les lave successivement avec 100 ml d'eau et 200 ml d'une solution saturée en chlorure de sodium. On les sèche sur du sulfate de magnésium, on évapore les solvants et on purifie le résidu par chromatographie sur colonne de gel de silice.
On obtient 15,7 g de produit sous forme d'un solide blanc.
Point de fusion = 143-145 °C Rendement = 61 %

### B 2'[2-(1,1-diméthyléthyl)-2H-tétrazol-5-yl][1,1'-biphényl]-4-carboxaldéhyde

Dans un ballon de 500 ml muni d'un réfrigérant, on introduit 9,2 g (21,3 mmoles) d'acide bis[2-[2-(1,1-diméthyléthyl)-2*H*-tétrazol-5-yl]phényl]borinique, 5 g (35,5 mmoles) de 4-chlorobenzaldéhyde, 2,05 g de palladiumtétrakis(triphénylphosphine), 36 ml d'une solution 2 M de carbonate de sodium et 150 ml de toluène. On porte le mélange réactionnel au reflux pendant 6 heures, puis on le verse dans 50 ml d'une solution saturée en chlorure d'ammonium. On extrait avec de l'acétate d'éthyle, on recueille la phase organique et on la lave successivement avec 100 ml d'eau et 100 ml d'une solution saturée en chlorure de sodium. On la sèche sur du sulfate de magnésium et on évapore sous vide. On purifie le résidu obtenu par chromatographie sur colonne de gel de silice.
On obtient 7,06 g de produit.
Point de fusion = 71-73 °C Rendement = 65 %

### C 2-(1,1-diméthyléthyl)-5-[2-(naphtalèn-1-yl)phényl]-2H-tétrazole

Dans un ballon tricol muni d'un réfrigérant, on introduit 4 g (9,3 mmoles) d'acide bis[2-[2-(1,1-diméthyléthyl)-2*H*-tétrazol-5-yl]phényl]borinique, 2,4 ml (16,7 mmoles) de 1-bromonaphtalène, 0,98 g de palladiumtétrakis(triphénylphosphine), 17 ml d'une solution 2 M de carbonate de sodium et 50 ml de toluène. On porte le mélange réactionnel au reflux pendant 10 heures, puis on le verse dans 50 ml d'une solution saturée en chlorure de sodium. On extrait avec de l'acétate d'éthyle, on recueille la phase organique et on la lave successivement avec 100 ml d'eau et 100 ml d'une solution saturée en chlorure de sodium. On la sèche sur du sulfate de magnésium et on évapore sous vide. On purifie le résidu obtenu par chromatographie sur colonne de gel de silice.
On obtient 3,6 g de produit.
Point de fusion = 83-85 °C Rendement = 65 %

### D 2-[2-[2-(1,1-diméthyléthyl)-2H-tétrazol-5-yl]phényl]-6-méthylquinoléine

Dans un ballon de 100 ml maintenu sous azote, on introduit 5,9 g (13,6 mmoles) d'acide bis[2-[2-(1,1-diméthyléthyl)-2*H*-tétrazol-5-yl]phényl]borinique, 3,84 g (21,6 mmoles) de 2-chloro-6-méthylquinoléine, 4,84 g de carbonate de sodium, 30 ml de toluène, 12 ml d'eau et 0,5 g de palladiumtétrakis(triphénylphosphine). On porte ce mélange à la température de reflux pendant 7 heures puis on le laisse revenir à la température ambiante. On laisse décanter le mélange puis on extrait la phase organique successivement par 20 ml puis par 10 ml d'une solution d'acide chlorhydrique 12 N. On lave la phase acide par 30 ml de toluène puis on ajuste le pH à 10 avec une solution de soude 10 N. On obtient un précipité gommeux que l'on reprend par de l'acétate d'éthyle puis on lave la phase organique avec de l'eau jusqu'à neutralité. On évapore le solvant à sec et on obtient un solide blanc que l'on recristallise dans 15 ml de tertbutylméthyléther. Finalement on filtre le précipité et on le sèche sous vide.
On obtient 5,67 g de produit sous forme d'un solide de couleur crème.
Point de fusion = 103-105 °C Rendement = 77 %

Le procédé selon l'invention permet d'obtenir des composés de formule (I) de haute pureté avec un bon rendement.
Il permet le couplage des halogénures aromatiques ou hétérocycliques comportant des fonctions organiques usuelles telles que par exemple les alcools, les éthers, les amines, les aldéhydes, les cétones, les acides, les esters, les nitriles, les amides, et les dérivés nitrés ou soufrés.
D'autre part ce procédé évite l'emploi d'azotures explosifs et contribue à la protection de l'environnement (recyclage du palladium).

## Revendications

1. Dérivés de l'acide benzèneborinique répondant à la formule (1) 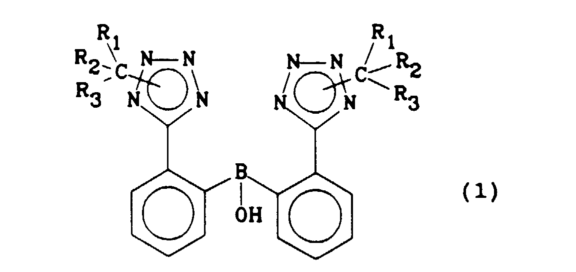 dans laquelle R₁, R₂ et R₃ représentent chacun indépendamment l'un de l'autre, soit un groupe (C₁-C₂)alkyle, soit un groupe aryle, le groupe -CR₁R₂R₃ étant en position 1 ou 2 du cycle tétrazole.

2. Dérivés de l'acide benzèneborinique selon la revendication 1 pour lesquels R₁, R₂, R₃ représentent chacun un groupe méthyle.

3. L'acide bis[2-[2-(1,1-diméthyléthyl)-2*H*-tétrazol-5-yl] phényl]borinique.

4. Procédé de préparation des dérivés selon la revendication 1, procédé caractérisé en ce que l'on fait réagir le 2-(1,1-diméthyléthyl)-5-phényltétrazole avec un alkyllithium dans un solvant aprotique à une température comprise entre - 50 °C et + 66 °C pour obtenir un organolithien que l'on fait réagir avec du trialkylborate dans un solvant aprotique pour obtenir un benzèneborinate d'alkyle que l'on soumet à une réaction d'hydrolyse.

5. Procédé de préparation des dérivés selon la revendication 1, procédé caractérisé en ce que l'on fait réagir un dérivé halogéné du 2-(1,1-diméthyléthyl)-5-phényltétrazole avec du magnésium dans un solvant aprotique pour obtenir un organomagnésien que l'on fait réagir avec du trialkylborate dans un solvant aprotique pour obtenir un benzèneborinate d'alkyle que l'on soumet à une réaction d'hydrolyse.

6. Utilisation des dérivés selon la revendication 1 pour la synthèse des composés répondant à la formule (I) 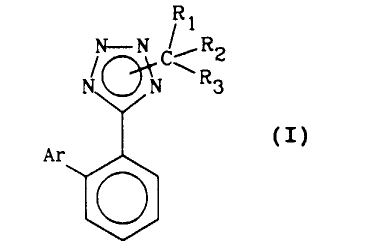 dans laquelle Ar représente soit un groupe aryle éventuellement substitué, soit un groupe naphtyle éventuellement substitué, soit un hétérocycle choisi parmi les groupes pyridinyle, pyrimidinyle, thiényle, imidazolyle, quinoléinyle et imidazopyridinyle éventuellement substitués et R₁, R₂ et R₃ sont tels que définis dans la revendication 1.

## Patentansprüche

1. Benzolborinsäure-Derivate der Formel (1) 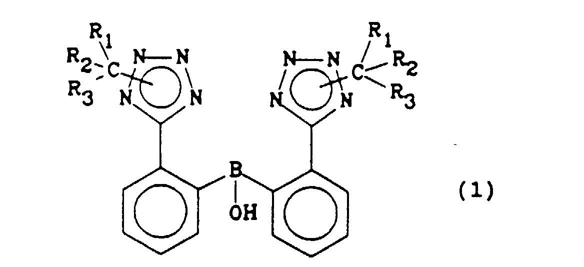 in der R₁, R₂ und R₃ jeweils unabhängig voneinander entweder eine (C₁-C₂)-Alkylgruppe oder eine Arylgruppe bedeuten, wobei die Gruppe -CR₁R₂R₃ in der 1-oder 2-Stellung des Tetrazolrings steht.

2. Benzolborinsäure-Derivate nach Anspruch 1, worin R₁, R₂ und R₃ jeweils eine Methylgruppe bedeuten.

3. Bis[2-[2-(1,1-Dimethylethyl)-2H-tetrazol-5-yl]-phenyl]-borinsäure.

4. Verfahren zur Herstellung der Derivate nach Anspruch 1, dadurch gekennzeichnet, daß man 2-(1,1-Dimethylethyl)-5-phenyltetrazol in einem aprotischen Lösungsmittel bei einer Temperatur zwischen -50°C und +66°C mit einem Alkyllithium umsetzt zur Bildung einer lithiumorganischen Verbindung, welche man in einem aprotischen Lösungsmittel mit Trialkylborat zur Bildung eines Benzolborinsäurealkylesters umsetzt, welchen man einer Hydrolysereaktion unterwirft.

5. Verfahren zur Herstellung der Derivate nach Anspruch 1, dadurch gekennzeichnet, daß man ein 2-(1,1-Dimethylethyl)-5-phenyltetrazol-Halogenderivat in einem aprotischen Lösungsmittel mit Magnesium zur Bildung einer magnesiumorganischen Verbindung umsetzt, welche man in einem aprotischen Lösungsmittel mit Trialkylborat umsetzt zur Bildung eines Benzolborinsäurealkylesters, den man einer Hydrolysereaktion unterwirft.

6. Verwendung der Derivate nach Anspruch 1 für die Herstellung von Verbindungen der Formel (I) 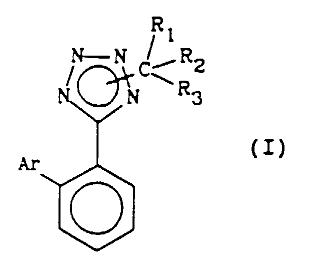 in der Ar entweder eine gegebenenfalls substituierte Arylgruppe oder eine gegebenenfalls substituierte Naphthylgruppe oder eine heterocyclische Gruppe ausgewählt aus gegebenenfalls substituierten Pyridinyl-, Pyrimidinyl-, Thienyl-, Imidazolyl-, Chinolinyl- und Imidazopyridinyl-gruppen umfassenden Gruppe bedeuten und R₁, R₂ und R₃ die in Anspruch 1 angegebenen Bedeutungen besitzen.

## Claims

1. Benzeneborinic acid derivatives corresponding to the formula (1) 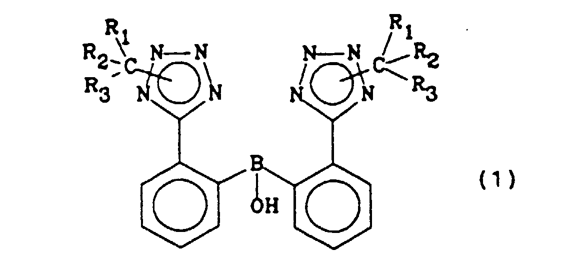 in which R₁, R₂ and R₃ each represent, independently of one another, either a (C₁-C₂)alkyl group or an aryl group, the group -CR₁R₂R₃ being in position 1 or 2 of the tetrazole ring.

2. Benzeneborinic acid derivatives according to Claim 1, for which R₁, R₂, R₃ each represent a methyl group.

3. Bis[2-[2-(1,1-dimethylethyl)-2*H*-tetrazol-5-yl]phenyl]borinic acid.

4. Process for preparing the derivatives according to Claim 1, which process is characterized in that 2-(1,1-dimethylethyl)-5-phenyltetrazole is reacted with an alkyllithium in an aprotic solvent at a temperature between -50°C and +66°C in order to obtain an organolithium compound which is reacted with trialkyl borate in an aprotic solvent in order to obtain an alkyl benzeneborinate which is subjected to a hydrolysis reaction.

5. Process for preparing the derivatives according to Claim 1, which process is characterized in that a halogenated derivative of 2-(1,1-dimethylethyl)-5-phenyltetrazole is reacted with magnesium in an aprotic solvent in order to obtain an organomagnesium compound which is reacted with trialkyl borate in an aprotic solvent in order to obtain an alkyl benzeneborinate which is subjected to a hydrolysis reaction.

6. Use of the derivatives according to Claim 1, for the synthesis of the compounds corresponding to the formula (I) 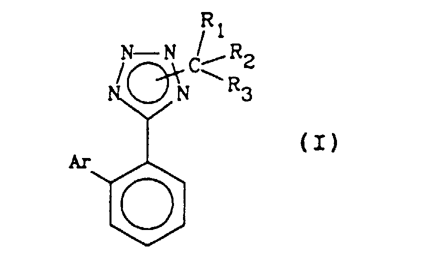 in which Ar represents either an optionally substituted aryl group or an optionally substituted naphthyl group, or a heterocycle chosen from optionally substituted pyridyl, pyrimidinyl, thienyl, imidazolyl, quinolyl and imidazopyridyl groups and R₁, R₂ and R₃ are as defined in Claim 1.
